**Europäisches Patentamt**

**European Patent Office**

**Office européen des brevets**

(19)

(11) Veröffentlichungsnummer: **0 003 105**
**B1**

(12) # EUROPÄISCHE PATENTSCHRIFT

(45) Veröffentlichungstag der Patentschrift: **20.05.81**

(21) Anmeldenummer: **79100017.7**

(22) Anmeldetag: **04.01.79**

(51) Int. Cl.³: **C 07 C 102/06,**
**C 07 D 307/68,**
**C 07 D 213/81,**
**C 07 C 103/76,**
**C 07 C 103/737,**
**C 07 D 307/54,**
**C 07 D 309/28,**
**C 07 D 333/40**

(54) Verfahren zur Anilidierung von Carbonsäureestern.

(30) Priorität: **05.01.78 DE 2800506**

(43) Veröffentlichungstag der Anmeldung:
**25.07.79 Patentblatt 79/15**

(45) Bekanntmachung des Hinweises auf die Patenterteilung:
**20.05.81 Patentblatt 81/20**

(84) Benannte.Vertragsstaaten:
**BE CH DE FR GB IT NL SE**

(56) Entgegenhaltungen:
DE - C - 347 607
US - A - 2 752 355

CHEMICAL ABSTRACTS, Vol. 19, 20. Februar
1925, Nr. 4, Columbus, Ohio, USA,
A. TERENTIEFF: "Magnesium anilide", Seite 641

CHEMICAL ABSTRACTS, Vol. 55, 12. Juni 1961,
Nr. 12, Columbus, Ohio, USA,
JUNJI FURUKAWA et al.: "Synthesis of methacrylanilide with aluminum anilide", Spalte
11362

CHEMICAL ABSTRACTS, Vol. 56, 19.
Februar 1962, Nr. 4, Columbus, Ohio, USA,
JUNJI FURUKAWA et al.: "Reactions of
aluminum anilide on some unsaturated esters and
nitriles", Spalte 3498i

JOURNAL OF THE CHEMICAL SOCIETY, 1954,
H.L1. BASSETT et al.: The bodroux reaction"

(73) Patentinhaber: WACKER-CHEMIE GMBH
Prinzregentenstrasse 22
D-8000 München 22 (DE)

(72) Erfinder: Deinhammer, Wolfgang, Dr. Dipl.-Chem.
Röntgenstrasse 32
D-8263 Burghausen (DE)
Erfinder: Spes, Hellmuth, Dr. Dipl.-Chem.
Nikolaus-Otto-Strasse 2
D-8263 Burghausen (DE)

EP 0 003 105 B1

## Verfahren zur Anilidierung von Carbonsäureestern

Die direkte Umsetzung von Carbonsäureestern mit Anilin führt häufig nicht zu technisch vertretbaren Ausbeuten und Reinheiten. Die Anilidierung vermittels Natriumanilid erfordert den technisch problematischen Umgang mit Natrium bzw. Natriumhydrid zur Herstellung des Natriumanilids. Die Verfahrensvariante, Carbonsäureanilide aus Carbonsäureestern und Halogenmagnesiumanilid zu erhalten, beinhaltet den Nachteil, Grignard-Reaktiönen im technischen Maßstab einsetzen zu müssen. Zudem sind pro Mol Reaktionsprodukt 2 Mole der Grignard-Verbindung erforderlich.

Weiterhin beschreiben Terentieff et al in C.A., Vol. 19, Nr. 4, die Anilidierungsreaktion von Essigsäureethylester mit Magnesiumanilid. Es handelt sich hierbei jedoch um eine Gasphasenreaktion unter vergleichsweise drastischen, für die im folgenden beschriebenen, erfindungsgemäß herzustellenden Verbindungen sich verbietenden Reaktionsbedingungen.

Ferner ist gemäß DE—PS 347 607 die Umsetzung von beispielsweise Magnesiumanilid mit aromatischen Oxycarbonsäuren zu den entsprechenden Aniliden bekannt. Bei diesen Reaktionen muß jedoch ein großer Überschuß an Magnesiumanilid eingesetzt werden, da ein erheblicher Teil durch Salzbildung verbraucht wird.

Aufgabe der Erfindung ist es, einen gegenüber dem Stand der Technik einfacheren Reaktionsweg vorzuschlagen, der zudem gegenüber herkömmlichen Herstellungsverfahren größere Ausbeuten und leichter auarbeitbare Reaktionsprodukte liefert.

Gegenstand der Erfindung ist eine Verfahren zur Anilidierung von Carbonsäureestern der allgemeinen Formel $R_1COOR_2$, das dadurch gekennzeichnet ist, daß solche Carbonsäureester, in denen

$R_1$ einen gegebenenfalls substituierten cycloaliphatischen Rest mit 3 bis 8 Kohlenstoffatomen, einen gegebenenfalls substituierten aromatischen Rest oder einen gegebenenfalls substituierten heterocyclischen Rest bedeutet, wobei die Substituenten Alkylgruppen mit bis zu 4 Kohlenstoffatomen bzw. bis zu 2 weitere der Anilidierung unterliegende Carboxylgruppen sein können,

$R_2$ ein Kohlenwasserstoffrest mit bis zu 4 Kohlenstoffatomen sein kann und

in Gegenwart von Anilin mit äquimolaren Mengen Magnesiumdianilid in flüssiger Phase umgesetzt werden.

Vorzugsweise werden solche Carbonsäureester umgesetzt, in denen $R_1$ für einen, gegebenenfalls substituierten, heterocyclischen Rest mit 4 bis 5 Ringkohlenstoffatomen steht.

Es ist besonders bevorzugt, solche Carbonsäureester umzusetzen, in denen $R_1$ für einen, gegebenenfalls substituierten, sauerstoffhaltigen heterocyclischen Rest mit 4 bis 5 Kohlenstoffringatomen steht.

Weiterhin ist es bevorzugt, solche Carbonsäureester umzusetzen, für die $R_2$ einen Kohlenwasserstoffrest mit bis zu 2 Kohlenstoffatomen bedeutet.

Vorteilhaft ist es, in bis zu 20-fachem molarem Überschuß von Anilin, bezogen auf Carbonsäureester, zu arbeiten. Manchmal kann es von Vorteil sein, in Gegenwart von organischen Lösungsmitteln zu arbeiten.

Überraschenderweise wird bei der erfindungsgemäßen Umsetzung gegenüber herkömmlichen Verfahren eine höhere Ausbeute mit Reinheiten des Reaktionsproduktes über 96 Gew.% erzielt, so daß ein weiterer Reinigungsschritt, beispielsweise durch Umkristallisieren, häufig entfallen kann. Dies ist umso erstaunlicher, als der Fachmann bei Kenntnis der Veröffentlichung von Lazier und Atkins, J. Am. Chem. Soc. 46, 741 bis 743, befürchten mußte, daß das während der Umsetzung entstehende Magnesiumalkoholat ein schwer zu entfernendes, alkyliertes Anilid ergeben würde. Außerdem werden durch das erfindungsgemäße Verfahren Anilidierungen von Carbonsäureestern möglich, die bei herkömmlicher Verfahrensweise nich oder nur schwer durchführbar sind.

Die Herstellung des erfindungsgemäß eingesetzten Magnesiumanilids erfolgt durch Erhitzen einer Mischung von Magnesium und Anilin. Dabei wird das Metall in Form von Spänen eingesetzt. Bei Anilinüberschuß fällt das Metallanilid in Lösung bzw. Suspension an.

In diese Lösung bzw. Suspension von Magnesiumdi- anilid wird unter Rühren der Carbonsäureester eingebracht. Wegen der auftretenden Reaktionswärme ist es mitunter notwendig zu kühlen. Anschließend an die Hauptreaktion ist es zweckmäßig, die Reaktionsmischung bei der Reaktionstemperatur 10 Minuten bis 24 Stunden zu belassen. Als Reaktionstemperatur der Umsetzung, die bei Normaldruck durchgeführt wird, sei 20 bis 180° C genannt, vorzugsweise wird bei 50 bis 140° C gearbeitet. Die Zugabemenge des Carbonsäureesters ist quimolar, bezogen auf Metallanilid. Bei mehreren zu anilidierenden Carbonsäureestergruppen in einem Molekül wird entsprechend verfahren. Natürlich kann als Verdünnungsmittel dem Reaktionsgemisch ein inerter Kohlenwasserstoff, wie beispielsweise Benzol, Toluol, Xylol, Cyclohexan oder Äther, wie Diäthyläther, Diisopropyläther, Dibutyläther, zugegeben werden. Vorzugsweise wird die Reaktion jedoch ausschließlich in überschüssigem Anilin aus der Metallanilidbildung durchgeführt. Die Abtrennung des überschüssigen Anilins nach der Reaktion bereitet keine Schwierigkeiten. Vorzugsweise wird ein Großteil des Anilins wegen seines, gegenüber dem Carbonsäureanilid stets niedrigeren Siedepunktes, abdestilliert, vorzugsweise bei vermindertem Druck.

Das verbleibende Gemisch kann mit Wasser aufgearbeitet werden, wobei sich Alkohol, Carbonsäureanilid und Metallhydroxid bildet. Danach wird mit Mineralsäure, vorzugsweise Salzsäure oder Schwefelsäure, angesäuert und das in wäßrigem Medium unlösliche Carbonsäureanilid abfiltriert. Eine Aufarbeitungsvariante besteht darin, das Anilid in organischen Lösungsmitteln, die mit Wasser eine Phasentrennung ergeben, ggf. in der Wärme aufzulösen, die Lösung mit Wasser zu versetzen, mit Mineralsäure anzusäuern und nach Trennung der wäßrigen von der organischen Phase die organische Phase aufzuarbeiten. Der verbleibende Rückstand kann umkristallisiert werden. Meist ist die Reinheit des Produktes jedoch so hoch, daß das Produkt sogleich als Handelsprodukt konfektioniert werden kann.

Als Beispiele für Verbindungen, die nach dem erfindungsgemäßen Verfahren u.a. hergestellt werden können, werden genannt:

1. Aromatische Anilide, wie 2-Methylbenzanilid oder 1, 3, 5-Benzoltricarbonsäureanilid
2. Gesättigte einkernige cycloaliphatische Anilide sowie einfach ungesättigte einkernige cycloaliphatische Anilide, wie 2-Methyl-cyclohex-1-en-1-carboxanilid
3. Gesättigte oder teilwise ungesättigte heterocyclische Systeme, 2,3-Dihydro-4H-thiopyrananilide, wie 2-Methyl-5,6-Dihydro-4H-thiopyran-3-carboxanilid, Dihydro-p-dioxinanilide sowie 2,3-Dihydro-1,4-oxathinanilide, vorzugsweise 2,3-Dihydrofurananilide, 2,3-Dihydro-4H-pyrananilide, insbesondere 2-Methyl-4,5-dihydrofuran-3-carboxanilid, 2-Methyl-5,6-dihydro-4H-pyran-3-carboxanilid
4. Ungesättigte (aromatische) heterocyclische Anilide, wie beispielsweise Thiophen-2-carboxanilid, 2-Methyl-thiophen-3-carboxanilid, 2-Methylpyridin-3-carboxanilid oder 2,6-Dimethyl-pyridin-3,5-dicarboxananilid, vorszugsweise enthaltend den Furanring, insbesondere 2-Methyl-furan 3-carboxanilid oder 2,5-Dimethyl-3-furan-carboxanilid oder 2,4,5-Trimethyl-3-furan-3-carboxanilid.

Die nach dem erfindungsgemäßen Verfahren hergestellten Carbonsäureanilide können als Zwischenprodukte dienen. Einige Vertreter dieser Verbindungsklasse sind wertvolle Pflanzenschutzmittel.

## Beispiel 1
### 2-Methyl-3-furancarboxanilid

25 g Magnesiumspäne, 0,1 g Natriumhydrid (80 %-ig in Weißöl) und 600 ml Anilin wurden 7 Stunden unter Ausschluß von Luftsauerstoff unter Rückfluß erhitzt, wobei Wasserstoffentwicklung auftrat, dann auf 120° abgekühlt und 140 g 2-Methyl-furan-3-carbonsäuremethylester innerhalb von 20 Minuten unter Rühren zugetropft. Nach zweistündigem Rühren bei 110 bis 120° wurde das überschüssige Anilin im Vakuum abdestilliert. Die verbleibende Schmelze wurde in 400 ml Toluol bei 80° gelöst. Dann wurde bei 60 bis 80° 100 ml Wasser und anschließend 350 ml 30 %-ige Salzsäure zugegeben. Die untere wäßrige Schicht wurde abgetrennt und kann zur Gewinnung des als Hydrochlorid gelösten Anilins durch Alkalischmachen weiterverarbeitet werden.

Die toluolische Lösung wurde noch zweimal bei 60 bis 80° mit 5%-iger Salzsäure, dann zweimal mit Wasser, angeschüttelt und ergab nach dem Abdampfen des Toluols 196 g 2-Methyl-3-furancarboxanilid, Reinheit 98 %-ig.

## Beispiel 2
### 2,5-Dimethyl-3-furancarboxanilid

Aus 25 g Magnesiumpänen in 600 ml Anilin und 168 g 2,5-Dimethyl-3-furancarbonsäureäthylester wurden 208 g 2,5-Dimethyl-3-furancarboxananilid mit einer Reinheit von 97,5% nach Beispiel 2 gewonnen.

## Beispiele 3 bis 6

Aus 1 Mol Magnesium und 1 Mol Carbonsäureäthylester wurden nach Beispiel 2 folgende Anilide in Ausbeuten von 95 bis 98% und Reinheiten von 96 bis 99% hergestellt:

Beispiel 4: 2-Thiophencarboxanilid
Beispiel 5: 2-Methyl-4,5-dihydrofuran-3-carboxanilid
Beispiel 6: 2-Methyl-5,6-dihydro-4H-pyran-3-carboxananilid
Beispiel 7: 2-Methyl-cyclohex-1-en-1-carboxanilid

## Beispiel 7

Zu 0,1 Mol Magnesiumanilid in 100 ml Anilin wurden bei 120° 12,5 g 2,6-Dimethyl-3,5-pyridindicarbonsäureäthylester innerhalb von 5 Minuten zugetropft und bei 110° 5 Stunden gerührt. Zu dem Reaktionsgemisch wurde 500 ml Wasser gegeben und mit konzentrierter Salzsäure unter Rühren auf pH

# 0 003 105

2 angesäuert. Dann wurde der entstehende Niederschlag abgesaugt, neutralgewaschen und getrocknet: 17,5 g 2,6-Dimethylpyridin-3,5-dicarboxanilid.

Die in den Beispielen 1 bis 7 hergestellten Anilide stimmen in ihren physikalischen Eigenschaften mit den nach bekannten Methoden hergestellten Präparaten überein.

## Patentansprüche

1. Verfahren zur Anilidierung von Carboansäureestern der allgemeinen Formel $R_1COOR_2$, dadurch gekennzeichnet, daß solche Carbonsäureester, in denen

$R_1$ einen gegebenenfalls substituierten cycloaliphatischen Rest mit 3 bis 8 Kohlenstoffatomen, einen gegebenenfalls substituierten aromatischen Rest oder einen gegebenenfalls substituierten heterocyclischen Rest bedeutet, wobei die Substituenten Alkylgruppen mit bis zu Kohlenstoffatomen bzw. bis zu 2 weitere der Anilidierung unterliegende Carboxylgruppen sein können,

$R_2$ ein Kohlenwasserstoffrest mit bis zu 4 Kohlenstoffatomen sein kann und in Gegenwart von Anilin mit äquimolaren Mengen Magnesiumdianilid in flüssiger Phase umgesetzt werden.

2. Verfahren nach Anspruch 1, dadurch gekennzeichnet, daß solche Carbonsäureester in denen $R_1$ für einen gegebenenfalls substituierten heterocyclischen Rest mit 4 bis Ringkohlenstoffatomen steht,
umgesetzt werden.

3. Verfahren nach Anspruch 2, dadurch gekennzeichnet, daß solche Carbonsäureester, in denen $R_1$ für einen gegebenenfalls substituierten, sauerstoffhaltigen heterocyclischen Rest mit 4 bis 5 Kohlenstoffringatomen steht,
umgesetzt werden.

4. Verfahren nach einem der Ansprüche 1, 2 oder 3, dadurch gekennzeichnet, daß solche Carbonsäureester, in denen
$R_2$ ein Kohlenwasserstoffrest mit bis zu 2 Kohlenstoffatomen sein kann,
umgesetzt werden.

5. Verfahren nach einem der Ansprüche 1, 2, 3 oder 4, dadurch gekennzeichnet, daß in Gegenwart bis zu zwanzigfachen Überschusses an Anilin, bezogen auf Carbonsäureester, bei Normaldruck umgesetzt wird.

## Claims

1. Process for the anilidation of carboxylic acid esters of the general formula $R_1COOR_2$, characterised in that those carboxylic acid esters in which

$R_1$ represents an optionally substituted cycloaliphatic radical having from 3 to 8 carbon atoms, an optionally substituted aromatic radical or an optionally substituted heterocyclic radical, wherein the substituents may be alkyl groups having up to 4 carbon atoms and may be up to 2 further carboxylic groups subject to anilidation, and

$R_2$ may be a hydrocarbon radical having up to 4 carbon atoms are reacted in the presence of aniline with equimolar amounts of magnesium dianilide in the liquid phase.

2. Process according to claim 1, characterised in that the reaction is carried out using those carboxylic acid esters in which

$R_1$ represents an optionally substituted heterocyclic radical having 4 or 5 ring carbon atoms.

3. Process according to claim 2, characterised in that the reaction is carried out using those carboxylic acid esters in which

$R_1$ represents an optionally substituted, oxygen-containing, heterocyclic radical having 4 or 5 ring carbon atoms.

4. Process according to any one of claims 1, 2 and 3, characterised in that the reaction is carried out using those carboxylic acid esters in which

$R_2$ may be a hydrocarbon radical having up to 2 carbon atoms.

5. Process according to any one of claims 1, 2, 3 and 4, characterised in that the reaction is carried out at normal pressure in the presence of up to a twenty-fold excess of aniline, calculated on the carboxylic acid ester.

## Revendications

1. Procédé d'anilidation d'esters d'acides carboxyliques répondant à la formule générale $R_1COOR_2$, procédé caractérisé en ce qu'on fait réagir des esters d'acides carboxyliques de ce genre dans lesquels:

$R_1$ représente un radical cyclo-aliphatique contenant de 3 à 8 atomes de carbone et éventuellement substitué, un radical aromatique éventuellement substitué ou un radical hétérocyclique éventuellement substitué, les substituants pouvant être des radicaux alkyles contenant au plus 4 atomes de carbone et/ou au plus deux groupes carboxy supplémentaires pouvant participer à l'anilidation, et

4

**0 003 105**

$R_2$ représente un radical hydrocarboné pouvant contenir jusqu'à 4 atomes de carbone, en présence d'aniline, avec des quantités équimolaires de dianilidure de magnésium, en phase liquide.

2. Procédé selon la revendication 1, caractérisé en ce qu'on fait réagir des esters d'acides carboxyliques dans lesquels $R_1$ représente un radical hétérocyclique éventuellement substitué, qui contient 4 ou 5 atomes de carbone dans son cycle.

3. Procédé selon la revendication 2, caractérisé en ce qu'on fait réagir des esters d'acides carboxyliques dans lesquels $R_1$ représente un radical hétérocyclique à 4 ou 5 atomes de carbone dans le cycle qui contient de l'oxygène et qui est éventuellement substitué.

4. Procédé selon l'une quelconque des revendications 1, 2 et 3, caractérisé en ce qu'on fait réagir des esters d'acides carboxyliques dans lesquels $R_2$ peut représenter un radical hydrocarboné contenant au plus 2 atomes de carbone.

5. Procédé selon l'une quelconque des revendications 1, 2, 3 et 4, caractérisé en ce qu'on effectue la réaction sous le pression normale en présence d'un excès d'aniline pouvant aller jusqu'à 20 fois la quantité molaire par rapport à l'ester d'acide carboxylique.